# EUROPEAN PATENT APPLICATION

(11) **EP 2 783 701 A2**
(43) Date of publication of application: **01.10.2014**
(21) Application number: 14165153.9
(22) Date of filing: 18.08.2005
(51) Int. Cl.: A61K 39/395, C07K 16/28, A61P 25/28, A61P 37/00, A61K 31/7056, A61K 38/21, A61K 31/225

(54) **Extended treatment of multiple sclerosis**

(30) Priority: 20.08.2004 US 603468 P; 20.08.2004 US 603495 P; 20.08.2004 US 603470 P; 05.10.2004 US 616023 P
(62) Divisional of application: 05804218.5
(71) Applicant: Biogen Idec MA Inc., Cambridge, Massachusetts 02142 (US)
(72) Inventor: Panzara, Michael, Winchester, MA 01890 (US); Toal, Martin, Stoke Poges, SL2 4EY (GB); Lynn, Frances, Somerville, MA 02143 (US)
(74) Representative: Miller, David James

(57) **Abstract**

Methods for extended treatment of multiple sclerosis are described.

## Description

### RELATED APPLICATIONS

This application claims the benefit of U.S. Provisional Application No. 60/603,468 filed August 20, 2004; of U.S. Provisional Application No. 60/603,495 filed August 20, 2004; of U.S. Provisional Application No. 60/603,470 filed August 20, 2004; and of U.S. Provisional Application No. 60/616,023 filed October 5, 2004; the entire contents of all of which are hereby incorporated by reference herein.

### BACKGROUND

Multiple sclerosis (MS) is one of the most common diseases of the central nervous system. Today over 2,500,000 people around the world have MS.

### SUMMARY

The invention is based, at least in part, on the finding that anti-VLA-4 therapy (e.g., anti-VLA-4 antibody therapy, e.g., natalizumab) is safe and effective for long-term administration to provide a therapeutic effect for multiple sclerosis (MS). Accordingly, in one aspect, the disclosure features a method of treating multiple sclerosis in a subject, e.g., a human subject. The method includes administering a therapeutically effective amount of a VLA-4 binding antibody to the subject for an extended duration.

In one embodiment, a therapeutically effective amount of the VLA-4 binding antibody is administered for at least 12 months, e.g., at least 18 months, preferably at least 24 months, e.g., at least 30, 36, 42, 48 months or longer. In one embodiment, a patient is selected on the basis of having previously had at least 21 doses of VLA-4 binding antibody, e.g., in a 24 month period (e.g., having had 24 monthly doses in the previous 2 years). The patient is then administered an additional course of VLA-4 binding antibody treatment to provide an improved therapeutic result, e.g., relative to before the commencement of VLA-4 binding antibody treatment, or relative to before the commencement of the additional course. An additional course can include, e.g., at least 3, e.g., at least 4, 5, 6, 8, 12, 16, 24, 30 or more, administrations of a therapeutically effective amount of a VLA-4 binding antibody.

In one embodiment, the patient can be administered a therapeutically effective amount of a VLA-4 binding antibody once a week, once a month, once every 6 weeks, or once every 2, 3, 4, or 6 months.

In one embodiment, a VLA-4 binding antibody is administered for at least 12 months and is effective to result in one or more of the following:
a) a decreased rate of relapse (e.g., at least 10%, 20%, 30%, 40%, 50%, 60%, 70%, 80% or greater reduction in rate of relapse) compared to the rate of relapse before the long-term administration (e.g., compared to the rate of relapse following administration for 12 months or for less than 12 months, e.g., less than 10, 8,4 or less months) of treatment, or before commencement of treatment, when measured between 3-24 months (e.g., between 6-18 months, e.g., 12 months) after a previous relapse;
b) prevention of an increase in EDSS score;
c) decreased EDSS score (e.g., a decrease of 1, 1.5, 2, 2.5, 3 points or more, e.g., over at least three months, six months, one year, or longer) compared to the EDSS score following administration for 12 months or for less than 12 months, e.g., less than 10, 8, 4 or less months, or before the commencement of treatment;
d) decreased number of new lesions overall or of any one type (e.g., at least 10%, 20%, 30%, 40% decrease), compared to the number of new lesions following administration for 12 months or for less than 12 months, e.g., less than 10, 8, 4 or less months, or before commencement of treatment;
e) decreased number of lesions overall or of any one type (e.g., at least 10%, 20%, 30%, 40% decrease), compared to the number of lesions following administration for 12 months or for less than 12 months, e.g., less than 10, 8, 4 or less months, or before commencement of treatment;
f) reduced rate of appearance of new lesions overall or of any one type (e.g., at least 10%, 20%, 30%, 40% reduced rate), compared to the rate of appearance of new lesions following administration for 12 months or for less than 12 months, e.g., less than 10, 8,4 or less months, or before commencement of treatment;
g) decreased increase in lesion area overall or of any one type (e.g., at least 10%, 20%, 30%, 40% decreased increase), compared to an increase in lesion area following administration for 12 months or less than 12 months, e.g., less than 10, 8,4 or less months, or before commencement of treatment; and
h) reduced incidence or symptom of optic neuritis (e.g., improved vision), compared to the incidence or symptom of optic neuritis following administration for 12 months or for less than 12 months, e.g., less than 10, 8,4 or less months, or before commencement of treatment.

In one embodiment, a VLA-4 binding antibody is administered for at least 18 months and is effective to result in one or more of the following:
a) a decreased rate of relapse (e.g., at least 10%, 20%, 30%, 40%, 50%, 60%, 70%, 80% or greater reduction in rate of relapse) compared to the rate of relapse before the long-term administration (e.g., compared to the rate of relapse following administration for 18 months or for less than 18 months, e.g., less than 16, 12, 8, 4 or less months) of treatment, or before commencement of treatment, when measured between 3-24 months (e.g., between 6-18 months, e.g., 12 months) after a previous relapse;
b) prevention of an increase in EDSS score;
c) decreased EDSS score (e.g., a decrease of 1, 1.5, 2, 2.5, 3 points or more, e.g., over at least three months, six months, one year, or longer) compared to the EDSS score following administration for 18 months or for less than 18 months, e.g., less than 16, 12, 8,4 or less months, or before the commencement of treatment;
d) decreased number of new lesions overall or of any one type (e.g., at least 10%, 20%, 30%, 40% decrease), compared to the number of new lesions following administration for 18 months or for less than 18 months, e.g., less than 16, 12, 8, 4 or less months, or before commencement of treatment;
e) decreased number of lesions overall or of any one type (e.g., at least 10%, 20%, 30%, 40% decrease), compared to the number of lesions following administration for 18 months or for less than 18 months, e.g., less than 16, 12, 8, 4 or less months, or before commencement of treatment;
f) reduced rate of appearance of new lesions overall or of any one type (e.g., at least 10%, 20%, 30%, 40% reduced rate), compared to the rate of appearance of new lesions following administration for 18 months or for less than 18 months, e.g., less than 16, 12, 8, 4 or less months, or before commencement of treatment;
g) decreased increase in lesion area overall or of any one type (e.g., at least 10%, 20%, 30%, 40% decreased increase), compared to an increase in lesion area following administration for 18 months or less than 18 months, e.g., less than 16, 12, 8, 4 or less months, or before commencement of treatment; and
h) reduced incidence or symptom of optic neuritis (e.g., improved vision), compared to the incidence or symptom of optic neuritis following administration for 18 months or for less than 18 months, e.g., less than 16, 12, 8, 4 or less months, or before commencement of treatment.

In one embodiment, a VLA-4 binding antibody is administered for at least 24 months and is effective to result in one or more of the following:
a) a decreased rate of relapse (e.g., at least 10%, 20%, 30%, 40%, 50%, 60%, 70%, 80% or greater reduction in rate of relapse) compared to the rate of relapse before the long-term administration (e.g., compared to the rate of relapse following administration for 24 months or for less than 24 months, e.g., less than 20, 16, 12, 8,4 or less months) of treatment, or before commencement of treatment, when measured between 3-24 months (e.g., between 6-18 months, e.g., 12 months) after a previous relapse;
b) prevention of an increase in EDSS score;
c) decreased EDSS score (e.g., a decrease of 1, 1.5, 2, 2.5, 3 points or more, e.g., over at least three months, six months, one year, or longer) compared to the EDSS score following administration for 24 months or for less than 24 months, e.g., less than 20, 16, 12, 8, 4 or less months, or before the commencement of treatment;
d) decreased number of new lesions overall or of any one type (e.g., at least 10%, 20%, 30%, 40% decrease), compared to the number of new lesions following administration for 24 months or for less than 24 months, e.g., less than 20, 16, 12, 8, 4 or less months, or before commencement of treatment;
e) decreased number of lesions overall or of any one type (e.g., at least 10%, 20%, 30%, 40% decrease), compared to the number of lesions following administration for 24 months or for less than 24 months, e.g., less than 20, 16, 12, 8,4 or less months, or before commencement of treatment;
f) reduced rate of appearance of new lesions overall or of any one type (e.g., at least 10%, 20%, 30%, 40% reduced rate), compared to the rate of appearance of new lesions following administration for 24 months or for less than 24 months, e.g., less than 20, 16, 12, 8, 4 or less months, or before commencement of treatment;
g) decreased increase in lesion area overall or of any one type (e.g., at least 10%, 20%, 30%, 40% decreased increase), compared to an increase in lesion area following administration for 24 months or less than 24 months, e.g., less than 20, 16, 12, 8, 4 or less months, or before commencement of treatment; and
h) reduced incidence or symptom of optic neuritis (e.g., improved vision), compared to the incidence or symptom of optic neuritis following administration for 24 months or for less than 24 months, e.g., less than 20, 16, 12, 8, 4 or less months, or before commencement of treatment.

In one embodiment, a VLA-4 binding antibody is administered for at least 36 months and is effective to result in one or more of the following:
a) a decreased rate of relapse (e.g., at least 10%, 20%, 30%, 40%, 50%, 60%, 70%, 80% or greater reduction in rate of relapse) compared to the rate of relapse before the long-term administration (e.g., compared to the rate of relapse following administration for 36 months or for less than 36 months, e.g., less than 30, 24, 20, 16, 12, 8, 4 or less months) of treatment, or before commencement of treatment, when measured between 3-24 months (e.g., between 6-18 months, e.g., 12 months) after a previous relapse;
b) prevention of an increase in EDSS score;
c) decreased EDSS score (e.g., a decrease of 1, 1.5, 2, 2.5, 3 points or more, e.g., over at least three months, six months, one year, or longer) compared to the EDSS score following administration for 36 months or for less than 36 months, e.g., less than 30, 24, 20, 16, 12, 8,4 or less months, or before the commencement of treatment;
d) decreased number of new lesions overall or of any one type (e.g., at least 10%, 20%, 30%, 40% decrease), compared to the number of new lesions following administration for 36 months or for less than 36 months, e.g., less than 30, 24, 20, 16, 12, 8,4 or less months, or before commencement of treatment;
e) decreased number of lesions overall or of any one type (e.g., at least 10%, 20%, 30%, 40% decrease), compared to the number of lesions following administration for 36 months or for less than 36 months, e.g., less than 30, 24, 20, 16, 12, 8, 4 or less months, or before commencement of treatment;
f) reduced rate of appearance of new lesions overall or of any one type (e.g., at least 10%, 20%, 30%, 40% reduced rate), compared to the rate of appearance of new lesions following administration for 36 months or for less than 36 months, e.g., less than 30, 24, 20, 16, 12, 8, 4 or less months, or before commencement of treatment;
g) decreased increase in lesion area overall or of any one type (e.g., at least 10%, 20%, 30%, 40% decreased increase), compared to an increase in lesion area following administration for 36 months or less than 36 months, e.g., less than 30, 24, 20, 16, 12, 8, 4 or less months, or before commencement of treatment; and
h) reduced incidence or symptom of optic neuritis (e.g., improved vision), compared to the incidence or symptom of optic neuritis following administration for 36 months or for less than 36 months, e.g., less than 30, 24, 20, 16, 12, 8, 4 or less months, or before commencement of treatment.

In one embodiment, a VLA-4 binding antibody is administered for at least 48 months is effective to result in one or more of the following:
a) a decreased rate of relapse (e.g., at least 10%, 20%, 30%, 40%, 50%, 60%, 70%, 80% or greater reduction in rate of relapse) compared to the rate of relapse before the long-term administration (e.g., compared to the rate of relapse following administration for 48 months or for less than 48 months, e.g., less than 42, 36, 30, 24, 20, 16, 12, 8, 4 or less months) of treatment, or before commencement of treatment, when measured between 3-24 months (e.g., between 6-18 months, e.g., 12 months) after a previous relapse;
b) prevention of an increase in EDSS score;
c) decreased EDSS score (e.g., a decrease of 1, 1.5, 2, 2.5, 3 points or more, e.g., over at least three months, six months, one year, or longer) compared to the EDSS score following administration for 48 months or for less than 48 months, e.g., less than 42, 36, 30, 24, 20, 16, 12, 8, 4 or less months, or before the commencement of treatment;
d) decreased number of new lesions overall or of any one type (e.g., at least 10%, 20%, 30%, 40% decrease), compared to the number of new lesions following administration for 48 months or for less than 48 months, e.g., less than 42, 36, 30, 24, 20, 16, 12, 8, 4 or less months, or before commencement of treatment;
e) decreased number of lesions overall or of any one type (e.g., at least 10%, 20%, 30%, 40% decrease), compared to the number of lesions following administration for 48 months or for less than 48 months, e.g., less than 42, 36, 30, 24, 20, 16, 12, 8, 4 or less months, or before commencement of treatment;
f) reduced rate of appearance of new lesions overall or of any one type (e.g., at least 10%, 20%, 30%, 40% reduced rate), compared to the rate of appearance of new lesions following administration for 48 months or for less than 48 months, e.g., less than 42, 36, 30, 24, 20, 16, 12, 8, 4 or less months, or before commencement of treatment;
g) decreased increase in lesion area overall or of any one type (e.g., at least 10%, 20%, 30%, 40% decreased increase), compared to an increase in lesion area following administration for 48 months or less than 48 months, e.g., less than 42, 36, 30, 24, 20, 16, 12, 8, 4 or less months, or before commencement of treatment; and
h) reduced incidence or symptom of optic neuritis (e.g., improved vision), compared to the incidence or symptom of optic neuritis following administration for 48 months or for less than 48 months, e.g., less than 42, 36, 30, 24, 20, 16, 12, 8, 4 or less months, or before commencement of treatment.

Generally the administration of a VLA-4 binding antibody can be a long-term administration that effects a reduction, amelioration, or delay in progression, of any symptom of the disorder, e.g., any of those described herein.

In one embodiment, the subject has relapsing remitting multiple sclerosis. In another embodiment, the subject has chronic progressive multiple sclerosis, e.g., primary-progressive (PP), secondary progressive, or progressive relapsing multiple sclerosis.

In one embodiment, the VLA-4 binding antibody is a full length antibody such as an IgG1, IgG2, IgG3, or IgG4. Typically the antibody is effectively human, human, or humanized. The VLA-4 binding antibody can inhibit VLA-4 interaction with a cognate ligand of VLA-4, e.g., VCAM-1. The VLA-4 binding antibody binds to at least the α chain of VLA-4, e.g., to the extracellular domain of the α4 subunit. For example, the VLA-4 binding antibody recognizes epitope B (e.g., B1 or B2) on the α chain of VLA-4. The VLA-4 binding antibody may compete with or have an epitope which overlaps with, natalizumab, HP1/2, or other VLA-4 binding antibody described herein for binding to VLA-4. In a preferred embodiment, the VLA-4 binding antibody includes natalizumab or at least the heavy chain and light chain variable domains of natalizumab.

In one embodiment, the VLA-4 binding antibody is not administered in combination with another biologic immunomodulatory therapy (e.g., is not administered in combination with interferon therapy).

Generally, the subject is administered a plurality of doses of the VLA-4 binding antibody. The plurality of doses can be a part of a "long-term" regimen. For example, the subject can be administered doses of the VLA-4 binding antibody for at least 12 months, e.g., at least 18 months, preferably at least 24 months, e.g., at least 30 months, 36 months, 42 months, 48 months or longer.

In one embodiment, the VLA-4 binding antibody is administered at a dose sufficient to achieve at least 80% (preferably 90%, 95%, 100%, 110%, 120%, 130%, 140%, 150%, 160%, 180%, 200% or greater) of the bioavailability achieved with a monthly (e.g., once every four weeks) dose of between about 50 and 600 mg (e.g., between about 200 and 400 mg, e.g., about 300 mg by intravenous route). In one embodiment, the VLA-4 binding antibody is administered at a concentration effective to provide at least 50% (at least 60, 65, 70, 75, 80, 85, 90%) alpha4 integrin receptor saturation in the subject. For example, the VLA-4 binding antibody is administered as a monthly IV infusion of between about 50 and 600 mg (e.g., between about 200 and 400 mg, e.g., about 300 mg). In another example, the VLA-4 binding antibody is administered as a once weekly subcutaneous (SC) injection of between 25-300 mg (e.g., between 50 and 150 mg, e.g., about 75 mg).

The VLA-4 binding antibody can be administered in an amount that is effective to result in one or more of the following: a) decreased severity or frequency of relapse, b) prevention of an increase in EDSS score, c) decreased EDSS score (e.g., a decrease of 1, 1.5, 2, 2.5, 3 points or more, e.g., over at least three months, six months, one year, or longer), d) decreased number of new lesions overall or of any one type, e) reduced rate of appearance of new lesions overall or of any one type, and f) decreased increase in lesion area overall or of any one type. Generally the VLA-4 binding antibody can be administered in an amount that effects a reduction, amelioration, or delay in progression, of any symptom of the disorder, e.g., any of those described herein.

The subject can be evaluated, e.g., before, during or after receiving the VLA-4 binding antibody, e.g., for indicia of responsiveness. A skilled artisan can use various clinical or other indicia of effectiveness of treatment, e.g., EDSS score; MRI scan; relapse number, rate, or severity; multiple sclerosis functional composite (MSFC); multiple sclerosis quality of life inventory (MSQLI). The subject can be monitored at various times during a regimen. In one embodiment, the subject is not examined for interferon bioavailability (e.g., before or after the administering).

In one embodiment, the subject can be treated with a corticosteroid, e.g. a system corticosteroid, within five, ten, 30, or 60 days, prior to initially administering the VLA-4 binding antibody. In another embodiment, the subject can be treated with an immunosuppressive or immunomodulating treatment (e.g., interferon beta) within three months prior to initially administering the VLA-4 binding antibody. In another embodiment, the subject can be treated with glatiramer acetate within three months prior to initially administering the VLA-4 binding antibody.

In some embodiments, the VLA-4 binding antibody can be administered in combination with a second agent, e.g., a therapeutic biologic agent, to provide a combinatorial therapeutic effect. As used herein, "administered in combination" means that two or more agents are administered to a subject at the same time or within an interval, such that there is overlap of an effect of each agent on the patient. Preferably the administration of the first and second agent is spaced sufficiently close together such that a combinatorial effect is achieved. The interval can be an interval of hours, days or weeks. Generally, the agents are concurrently bioavailable, e.g., detectable, in the subject. In a preferred embodiment at least one administration of one of the agents, e.g., the first agent, is made while the other agent, e.g., the second agent, is still present at a therapeutic level in the subject. In one embodiment the second agent is administered between an earlier and a later administration of the first agent. In other embodiments the first agent is administered between an earlier and a later administration of the second agent. In one embodiment at least one administration of one of the agents, e.g., the first agent, is made within 1, 7, 14, 30, or 60 days of the second agent.

A "combinatorial therapeutic effect" is an effect, e.g., an improvement, that is greater than one produced by either agent alone. The difference between the combinatorial therapeutic effect and the effect of each agent alone can be a statistically significant difference. In one embodiment, the second agent comprises a biologic immunomodulating agent, e.g., interferon beta, e.g., interferon beta-1a (e.g., AVONEX® or Rebif®) or interferon beta-1b (e.g., Betaseron®). For example, a VLA-4 binding antibody is administered in combination with AVONEX® to a patient having MS. The second agent can also be a protein of undefined sequence, e.g., a random copolymer of selected amino acids, e.g., glatiramer acetate.

### Definitions

The term "treating" refers to administering a therapeutically effective amount of a therapy. "Therapeutically effective amount" refers to a therapy in amount, manner, and/or mode effective to improve a condition, symptom, or parameter associated with a disorder or to prevent progression of a disorder, to either a statistically significant degree or to a degree detectable to one skilled in the art. An effective amount, manner, or mode can vary depending on the subject and may be tailored to the subject.

"Extended" and "long-term", as they relate to duration or length of time of administration, means administration for at least 12 months. For example, long-term or extended administration can be at least 18 months, preferably at least 24 months, e.g., at least 30, 36, 42, 48 months or longer.

A "course" of treatment refers to treatment for a given length of time with a given number of administrations. Treatment can include multiple courses of therapy. For example, treatment can include a first course of administration for at least 12 months, e.g., at least 18 months, preferably at least 24 months, e.g., at least 30, 36, 42, 48 months or longer, followed by one or more additional courses of administration.

The term "biologic" refers to a protein-based therapeutic agent. In a preferred embodiment the biologic is at least 10, 20, 30, 40, 50 or 100 amino acid residues in length.

A "VLA-4 binding agent" refers to any compound that binds to VLA-4 integrin with a K_{d} of less than 10⁻⁶ M. An example of a VLA-4 binding agent is a VLA-4 binding protein, e.g., an antibody such as natalizumab.

A "VLA-4 antagonist" refers to any compound that at least partially inhibits an activity of a VLA-4 integrin, particularly a binding activity of a VLA-4 integrin or a signaling activity, e.g., ability to transduce a VLA-4 mediated signal. For example, a VLA-4 antagonist may inhibit binding of VLA-4 to a cognate ligand of VLA-4, e.g., a cell surface protein such as VCAM-1, or to an extracellular matrix component, such as fibronectin or osteopontin. A typical VLA-4 antagonist can bind to VLA-4 or to a VLA-4 ligand, e.g., VCAM-1 or an extracellular matrix component, such as fibronectin or osteopontin. A VLA-4 antagonist that binds to VLA-4 may bind to either the α4 subunit or the β1 subunit, or to both. A VLA-4 antagonist may also interact with other α4 subunit containing integrins (e.g., α4β7) or with other β1 containing integrins. A VLA-4 antagonist may bind to VLA-4 or to a VLA-4 ligand with a K_{d} of less than 10⁻⁶, 10⁻⁷, 10⁻⁸, 10⁻⁹, or 10⁻¹⁰ M.

A VLA-4 antagonist can be a compound that includes a protein moiety or a compound that does not include a protein moiety. Examples of VLA-4 protein antagonists include antagonizing antibodies, such as natalizumab, and peptide antagonists. Examples of non-protein antagonists include small molecule antagonists. A "small molecule" is an organic molecule that has a molecular weight of less than 1000 Daltons.

As used herein, the term "antibody" refers to a protein that includes at least one immunoglobulin variable region, e.g., an amino acid sequence that provides an immunoglobulin variable domain or immunoglobulin variable domain sequence. For example, an antibody can include a heavy (H) chain variable region (abbreviated herein as VH), and a light (L) chain variable region (abbreviated herein as VL). In another example, an antibody includes two heavy (H) chain variable regions and two light (L) chain variable regions. The term "antibody" encompasses antigen-binding fragments of antibodies (e.g., single chain antibodies, Fab fragments, F(ab')₂ fragments, Fd fragments, Fv fragments, and dAb fragments) as well as complete antibodies, e.g., intact immunoglobulins of types IgA, IgG, IgE, IgD, IgM (as well as subtypes thereof). The light chains of the immunoglobulin may be of types kappa or lambda. In one embodiment, the antibody is glycosylated. An antibody can be functional for antibody-dependent cytotoxicity and/or complement-mediated cytotoxicity, or may be non-functional for one or both of these activities.

The VH and VL regions can be further subdivided into regions of hypervariability, termed "complementarity determining regions" ("CDR"), interspersed with regions that are more conserved, termed "framework regions" ("FR"). The extent of the FR's and CDR's has been precisely defined (see, Kabat, E.A., et al. (1991) Sequences of Proteins of Immunological Interest, Fifth Edition, US Department of Health and Human Services, NIH Publication No. 91-3242; and Chothia, C. et al. (1987) J. Mol. Biol. 196:901-917). Kabat definitions are used herein. Each VH and VL is typically composed of three CDR's and four FR's, arranged from amino-terminus to carboxyl-terminus in the following order: FR1, CDR1, FR2, CDR2, FR3, CDR3, FR4.

An "immunoglobulin domain" refers to a domain from the variable or constant domain of immunoglobulin molecules. Immunoglobulin domains typically contain two β-sheets formed of about seven β-strands, and a conserved disulphide bond (see, e.g., A. F. Williams and A. N. Barclay 1988 Ann. Rev Immunol. 6:381-405).

As used herein, an "immunoglobulin variable domain sequence" refers to an amino acid sequence that can form the structure of an immunoglobulin variable domain. For example, the sequence may include all or part of the amino acid sequence of a naturally-occurring variable domain. For example, the sequence may omit one, two or more amino- or carboxyl-terminal amino acids, internal amino acids, may include one or more insertions or additional terminal amino acids, or may include other alterations. In one embodiment, a polypeptide that includes an immunoglobulin variable domain sequence can associate with another immunoglobulin variable domain sequence to form a target binding structure (or "antigen binding site"), e.g., a structure that interacts with VLA-4.

The VH or VL chain of the antibody can further include all or part of a heavy or light chain constant region, to thereby form a heavy or light immunoglobulin chain, respectively. In one embodiment, the antibody is a tetramer of two heavy immunoglobulin chains and two light immunoglobulin chains. The heavy and light immunoglobulin chains can be connected by disulfide bonds. The heavy chain constant region typically includes three constant domains, CH1, CH2 and CH3. The light chain constant region typically includes a CL domain. The variable region of the heavy and light chains contains a binding domain that interacts with an antigen. The constant regions of the antibodies typically mediate the binding of the antibody to host tissues or factors, including various cells of the immune system (e.g., effector cells) and the first component (Clq) of the classical complement system.

One or more regions of an antibody can be human, effectively human, or humanized. For example, one or more of the variable regions can be human, effectively human, or humanized. For example, one or more of the CDRs, e.g., HC CDR1, HC CDR2, HC CDR3, LC CDR1, LC CDR2, and LC CDR3, can be human. Each of the light chain CDRs can be human. HC CDR3 can be human. One or more of the framework regions can be human, e.g., FR1, FR2, FR3, and FR4 of the HC or LC. In one embodiment, all the framework regions are human, e.g., derived from a human somatic cell, e.g., a hematopoietic cell that produces immunoglobulins or a non-hematopoietic cell. In one embodiment, the human sequences are germline sequences, e.g., encoded by a germline nucleic acid. One or more of the constant regions can be human, effectively human, or humanized. In another embodiment, at least 70, 75, 80, 85,90,92,95, or 98% of the framework regions (e.g., FR1, FR2, and FR3, collectively, or FR1, FR2, FR3, and FR4, collectively) or the entire antibody can be human, effectively human, or humanized. For example, FR1, FR2, and FR3 collectively can be at least 70, 75, 80, 85, 90, 92, 95, 98, or 99% identical to a human sequence encoded by a human germline segment.

An "effectively human" immunoglobulin variable region is an immunoglobulin variable region that includes a sufficient number of human framework amino acid positions such that the immunoglobulin variable region does not elicit an immunogenic response in a normal human. An "effectively human" antibody is an antibody that includes a sufficient number of human amino acid positions such that the antibody does not elicit an immunogenic response in a normal human.

A "humanized" immunoglobulin variable region is an immunoglobulin variable region that is modified such that the modified form elicits less of an immune response in a human than does the non-modified form, e.g., is modified to include a sufficient number of human framework amino acid positions such that the immunoglobulin variable region does not elicit an immunogenic response in a normal human. Descriptions of "humanized" immunoglobulins include, for example, US Pat. No. 6,407,213 and US Pat. No. 5,693,762. In some cases, humanized immunoglobulins can include a non-human amino acid at one or more framework amino acid positions.

All or part of an antibody can be encoded by an immunoglobulin gene or a segment thereof. Exemplary human immunoglobulin genes include the kappa, lambda, alpha (IgA1 and IgA2), gamma (IgG1, IgG2, IgG3, IgG4), delta, epsilon and mu constant region genes, as well as the myriad immunoglobulin variable region genes. Full-length immunoglobulin "light chains" (about 25 Kd or 214 amino acids) are encoded by a variable region gene at the amino-terminus (about 110 amino acids) and a kappa or lambda constant region gene at the carboxyl-terminus. Full-length immunoglobulin "heavy chains" (about 50 Kd or 446 amino acids), are similarly encoded by a variable region gene (about 116 amino acids) and one of the other aforementioned constant region genes, e.g., gamma (encoding about 330 amino acids).

The term "antigen-binding fragment" of a full length antibody refers to one or more fragments of a full-length antibody that retain the ability to specifically bind to a target of interest, e.g., VLA-4. Examples of binding fragments encompassed within the term "antigen-binding fragment" of a full length antibody include (i) a Fab fragment, a monovalent fragment consisting of the VL, VH, CL and CH1 domains; (ii) a F(ab')₂ fragment, a bivalent fragment including two Fab fragments linked by a disulfide bridge at the hinge region; (iii) a Fd fragment consisting of the VH and CH1 domains; (iv) a Fv fragment consisting of the VL and VH domains of a single arm of an antibody, (v) a dAb fragment (Ward et al., (1989) Nature 341:544-546), which consists of a VH domain; and (vi) an isolated complementarity determining region (CDR) that retains functionality. Furthermore, although the two domains of the Fv fragment, VL and VH, are coded for by separate genes, they can be joined, using recombinant methods, by a synthetic linker that enables them to be made as a single protein chain in which the VL and VH regions pair to form monovalent molecules known as single chain Fv (scFv). See *e.g.,* Bird et al. (1988) Science 242:423-426; and Huston et al. (1988) Proc. Natl. Acad. Sci. USA 85:5879-5883.

### DETAILED DESCRIPTION

Multiple sclerosis (MS) is a central nervous system disease that is characterized by inflammation and loss of myelin sheaths. The invention is based, in part, on the discovery that anti-VLA-4 therapy (e.g., anti-VLA-4 antibody therapy, e.g., natalizumab) is safe and effective for long-term administration to provide a therapeutic effect for MS.

For example, a therapeutically effective amount of a VLA-4 binding antibody is administered for at least 12 months, e.g., at least 18 months, preferably at least 24 months, e.g., at least 30, 36, 42, 48 months or longer, and is effective to provide an improved therapeutic result as measured by, e.g., an MS-associated parameter. Exemplary MS-associated parameters include number of MRI detectable images (e.g., number of Gd+ lesions, T1 lesions, or T2 lesions), EDSS score, number and/or frequency of MS-related incidents, e.g., relapses.
1. MRI Detectable Images: MRI gadolinium-enhancing lesions are an indicator of migration of inflammatory cells into the CNS. This migration is a key pathogenic mechanism of MS. Accordingly, patients can be evaluated for MRI gadolinium-enhancing lesions. MRI can also be used to detect the location and extent of lesions using T₂-weighted techniques. See, e.g., McDonald et al. Ann. Neurol. 36:14, 1994.
2. EDSS Scoring: EDSS grades clinical impairment due to MS (Kurtzke, Neurology 33:1444, 1983). Eight functional systems are evaluated for the type and severity of neurologic impairment. Briefly, patients are evaluated for impairment in the following systems: pyramidal, cerebella, brainstem, sensory, bowel and bladder, visual, cerebral, and other. The scale ranges from 0 (normal) to 10 (death due to MS). Other examples of scoring systems include: multiple sclerosis functional composite (MSFC) and multiple sclerosis quality of life inventory (MSQLI). In addition, other MS associated parameters can be based on a particular neurological examinations.
3. MS-related Incidents: Exemplary MS-related incidents include attacks, relapses and exacerbations. An attack is an episode characterized by the acute onset of one or more symptoms. A relapse is the occurrence of an acute episode of new or worsening symptoms of multiple sclerosis that lasts at least 24 hours after a stable period of at least 30 days, and is accompanied by an increase of at least one point in EDSS score, at least one point on two functional system scores, or at least two points on one functional system score. Exacerbations are defined as the appearance of a new symptom that is attributable to MS and accompanied by an appropriate new neurologic abnormality (IFNB MS Study Group, supra). Typically, the exacerbation lasts at least 24 hours and is preceded by stability or improvement for at least 30 days. Exacerbations are either mild, moderate, or severe according to changes in a Neurological Rating Scale (Sipe et al., Neurology 34:1368,1984).

### Evaluating Therapy

A subject treated according to a method described herein can be monitored during therapy, e.g., to determine efficacy of the VLA-4 binding antibody therapy.

For example, MRI can be used to evaluate a therapy, e.g., a therapy that includes a VLA-4 binding antibody. In one implementation, baseline MRIs are obtained prior to therapy. The same imaging plane and patient position are used for each subsequent study. Positioning and imaging sequences can be chosen to maximize lesion detection and facilitate lesion tracing. The same positioning and imaging sequences can be used on subsequent studies. The presence, location and extent of MS lesions can be determined by a radiologist. Areas of lesions can be outlined and summed slice by slice for total lesion area. Three analyses may be done: evidence of new lesions, rate of appearance of active lesions, percentage change in lesion area (Paty et al., Neurology 43:665, 1993). Improvement due to therapy can be established by a statistically significant improvement in an individual patient compared to baseline or in a treated group versus a placebo group.

Therapy can be deemed to be effective if there is a statistically significant difference in the rate or proportion of exacerbation-free or relapse-free patients between the treated group and the placebo group for either of these measurements. In addition, time to first exacerbation and exacerbation duration and severity may also be measured. A measure of effectiveness as therapy in this regard is a statistically significant difference in the time to first exacerbation or duration and severity in the treated group compared to control group. An exacerbation-free or relapse-free period of greater than one year, 18 months, or 20 months is particularly noteworthy.

Efficacy of a VLA-4 binding therapy can also be evaluated based on one or more of the following criteria: frequency of MBP reactive T cells determined by limiting dilution, proliferation response of MBP reactive T cell lines and clones, cytokine profiles of T cell lines and clones to MBP established from patients. Efficacy is indicated by decrease in frequency of reactive cells, a reduction in thymidine incorporation with altered peptide compared to native, and a reduction in TNF and IFN-α.

Clinical measurements include the relapse rate in one and two-year intervals, and a change in EDSS, including time to progression from baseline of 1.0 unit on the EDSS that persists for six months. On a Kaplan-Meier curve, a delay in sustained progression of disability shows efficacy. Other criteria include a change in area and volume of T2 images on MRI, and the number and volume of lesions determined by gadolinium enhanced images.

Exemplary symptoms associated with multiple sclerosis, which can be treated with the methods described herein, include: optic neuritis, diplopia, nystagmus, ocular dysmetria, internuclear ophthalmoplegia, movement and sound phosphenes, afferent pupillary defect, paresis, monoparesis, paraparesis, hemiparesis, quadraparesis, plegia, paraplegia, hemiplegia, tetraplegia, quadraplegia, spasticity, dysarthria, muscle atrophy, spasms, cramps, hypotonia, clonus, myoclonus, myokymia, restless leg syndrome, footdrop, dysfunctional reflexes, paraesthesia, anaesthesia, neuralgia, neuropathic and neurogenic pain, l'hermitte's, proprioceptive dysfunction, trigeminal neuralgia, ataxia, intention tremor, dysmetria, vestibular ataxia, vertigo, speech ataxia, dystonia, dysdiadochokinesia, frequent micturation, bladder spasticity, flaccid bladder, detrusor-sphincter dyssynergia, erectile dysfunction, anorgasmy, frigidity, constipation, fecal urgency, fecal incontinence, depression, cognitive dysfunction, dementia, mood swings, emotional lability, euphoria, bipolar syndrome, anxiety, aphasia, dysphasia, fatigue, Uhthoffs symptom, gastroesophageal reflux, and sleeping disorders. Mitigation or amelioration or one more of these symptoms in a subject can be achieved by the VLA-4 binding antibody therapy.

Most commonly, MS first manifests itself as a series of attacks followed by complete or partial remissions as symptoms mysteriously lessen, only to return later after a period of stability. This is called relapsing-remitting (RR) MS. Primary-progressive (PP) MS is characterized by a gradual clinical decline with no distinct remissions, although there may be temporary plateaus or minor relief from symptoms. Secondary-progressive (SP) MS begins with a relapsing-remitting course followed by a later primary-progressive course. Rarely, patients may have a progressive-relapsing (PR) course in which the disease takes a progressive path punctuated by acute attacks. PP, SP, and PR are sometimes lumped together and called chronic progressive MS.

A few patients experience malignant MS, defined as a swift and relentless decline resulting in significant disability or even death shortly after disease onset. This decline may be arrested or decelerated by administration of a VLA-4 binding antibody (e.g., natalizumab) described herein.

### Natalizumab and Other VLA-4 Binding Antibodies

Natalizumab, an α4 integrin binding antibody, inhibits the migration of leukocytes from the blood to the central nervous system. Natalizumab binds to VLA-4 on the surface of activated T-cells and other mononuclear leukocytes. It can disrupt adhesion between the T-cell and endothelial cells, and thus prevent migration of mononuclear leukocytes across the endothelium and into the parenchyma. As a result, the levels of proinflammatory cytokines can also be reduced.

Natalizumab can decrease the number of brain lesions and clinical relapses in patients with relapse remitting multiple sclerosis and relapsing secondary-progressive multiple sclerosis. Natalizumab can be safely administered to patients with multiple sclerosis when combined with interferon β-1a (IFNβ-1a) therapy. Other VLA-4 binding antibodies can have these or similar properties

Natalizumab and related VLA-4 binding antibodies are described, e.g., in US Pat. No. 5,840,299. Monoclonal antibodies 21.6 and HP1/2 are exemplary murine monoclonal antibodies that bind VLA-4. Natalizumab is a humanized version of murine monoclonal antibody 21.6 (see, e.g., US Pat. No. 5,840,299). A humanized version of HP1/2 has also been described (see, e.g., US Pat. No. 6,602,503). Several additional VLA-4 binding monoclonal antibodies, such as HP2/1, HP2/4, L25 and P4C2, are described, e.g., in US Pat. No. 6,602,503; Sanchez-Madrid et al., 1986 Eur. J. Immunol., 16:1343-1349; Hemler et al., 1987 J. Biol. Chem. 2:11478-11485; Issekutz and Wykretowicz, 1991, J. Immunol., 147: 109 (TA-2 mab); Pulido et al., 1991 J. Biol. Chem., 266(16):10241-10245; and US Pat. No. 5,888,507).

Some VLA-4 binding antibodies recognize epitopes of the α4 subunit that are involved in binding to a cognate ligand, e.g., VCAM-1 or fibronectin. Many such antibodies inhibit binding of VLA-4 to cognate ligands (e.g., VCAM-1 and fibronectin).

Many useful VLA-4 binding antibodies interact with VLA-4 on cells, e.g., lymphocytes, but do not cause cell aggregation. However, other anti-VLA-4 binding antibodies have been observed to cause such aggregation. HP1/2 does not cause cell aggregation. The HP1/2 monoclonal antibody (Sanchez-Madrid et al., 1986) has an extremely high potency, blocks VLA-4 interaction with both VCAM1 and fibronectin, and has the specificity for epitope B on VLA-4. This antibody and other B epitope-specific antibodies (such as B1 or B2 epitope binding antibodies; Pulido et al., 1991, supra) represent one class of VLA-4 binding antibodies that can be used in the methods described herein.

An exemplary VLA-4 binding antibody has one or more CDRs, e.g., all three HC CDRs and/or all three LC CDRs of a particular antibody disclosed herein, or CDRs that are, in sum, at least 80, 85, 90, 92, 94, 95, 96, 97, 98, 99% identical to such an antibody, e.g., natalizumab. In one embodiment, the H1 and H2 hypervariable loops have the same canonical structure as those of an antibody described herein. In one embodiment, the L1 and L2 hypervariable loops have the same canonical structure as those of an antibody described herein.

In one embodiment, the amino acid sequence of the HC and/or LC variable domain sequence is at least 70, 80, 85, 90, 92, 95, 97, 98, 99, or 100% identical to the amino acid sequence of the HC and/or LC variable domain of an antibody described herein, e.g., natalizumab. The amino acid sequence of the HC and/or LC variable domain sequence can differ by at least one amino acid, but no more than ten, eight, six, five, four, three, or two amino acids from the corresponding sequence of an antibody described herein, e.g., natalizumab. For example, the differences may be primarily or entirely in the framework regions.

The amino acid sequences of the HC and LC variable domain sequences can be encoded by a nucleic acid sequence that hybridizes under high stringency conditions to a nucleic acid sequence described herein or one that encodes a variable domain or an amino acid sequence described herein. In one embodiment, the amino acid sequences of one or more framework regions (e.g., FR1, FR2, FR3, and/or FR4) of the HC and/or LC variable domain are at least 70, 80, 85, 90, 92, 95,97, 98, 99, or 100% identical to corresponding framework regions of the HC and LC variable domains of an antibody described herein. In one embodiment, one or more heavy or light chain framework regions (e.g., HC FR1, FR2, and FR3) are at least 70, 80, 85, 90, 95, 96, 97, 98, or 100% identical to the sequence of corresponding framework regions from a human germline antibody.

Calculations of "homology" or "sequence identity" between two sequences (the terms are used interchangeably herein) are performed as follows. The sequences are aligned for optimal comparison purposes (e.g., gaps can be introduced in one or both of a first and a second amino acid or nucleic acid sequence for optimal alignment and non-homologous sequences can be disregarded for comparison purposes). The optimal alignment is determined as the best score using the GAP program in the GCG software package with a Blossum 62 scoring matrix with a gap penalty of 12, a gap extend penalty of 4, and a frameshift gap penalty of 5. The amino acid residues or nucleotides at corresponding amino acid positions or nucleotide positions are then compared. When a position in the first sequence is occupied by the same amino acid residue or nucleotide as the corresponding position in the second sequence, then the molecules are identical at that position (as used herein amino acid or nucleic acid "identity" is equivalent to amino acid or nucleic acid "homology"). The percent identity between the two sequences is a function of the number of identical positions shared by the sequences.

As used herein, the term "hybridizes under high stringency conditions" describes conditions for hybridization and washing. Guidance for performing hybridization reactions can be found in Current Protocols in Molecular Biology, John Wiley & Sons, N.Y. (1989), 6.3.1-6.3.6, which is incorporated by reference. Aqueous and nonaqueous methods are described in that reference and either can be used. High stringency hybridization conditions include hybridization in 6X SSC at about 45°C, followed by one or more washes in 0.2X SSC, 0.1% SDS at 65°C, or substantially similar conditions.

Antibodies can be tested for a functional property, e.g., VLA-4 binding, e.g., as described in US Pat. No. 6,602,503.

### Antibody Generation

Antibodies that bind to VLA-4 can be generated by immunization, e.g., using an animal. All or part of VLA-4 can be used as an immunogen. For example, the extracellular region of the α4 subunit can be used as an immunogen. In one embodiment, the immunized animal contains immunoglobulin producing cells with natural, human, or partially human immunoglobulin loci. In one embodiment, the non-human animal includes at least a part of a human immunoglobulin gene. For example, it is possible to engineer mouse strains deficient in mouse antibody production with large fragments of the human Ig loci. Using the hybridoma technology, antigen-specific monoclonal antibodies derived from the genes with the desired specificity may be produced and selected. See, e.g., XenoMouse™, Green et al. Nature Genetics 7:13-21 (1994), US 2003-0070185, US Pat. No. 5,789,650, and WO 96/34096.

Non-human antibodies to VLA-4 can also be produced, e.g., in a rodent. The non-human antibody can be humanized, e.g., as described in US Pat. No. 6,602,503, EP 239 400, US Pat. No. 5,693,761, and US Pat. No. 6,407,213.

EP 239 400 (Winter et al.) describes altering antibodies by substitution (within a given variable region) of their complementarity determining regions (CDRs) for one species with those from another. CDR-substituted antibodies can be less likely to elicit an immune response in humans compared to true chimeric antibodies because the CDR-substituted antibodies contain considerably less non-human components. (Riechmann et al., 1988, Nature 332, 323-327; Verhoeyen et al., 1988, Science 239, 1534-1536). Typically, CDRs of a murine antibody substituted into the corresponding regions in a human antibody by using recombinant nucleic acid technology to produce sequences encoding the desired substituted antibody. Human constant region gene segments of the desired isotype (usually gamma I for CH and kappa for CL) can be added and the humanized heavy and light chain genes can be co-expressed in mammalian cells to produce soluble humanized antibody.

Queen et al., 1989 and WO 90/07861 have described a process that includes choosing human V framework regions by computer analysis for optimal protein sequence homology to the V region framework of the original murine antibody, and modeling the tertiary structure of the murine V region to visualize framework amino acid residues that are likely to interact with the murine CDRs. These murine amino acid residues are then superimposed on the homologous human framework. See also US Pat. Nos. 5,693,762; 5,693,761; 5,585,089; and 5,530,101. Tempest et al., 1991, Biotechnology 9, 266-271, utilize, as standard, the V region frameworks derived from NEWM and REI heavy and light chains, respectively, for CDR-grafting without radical introduction of mouse residues. An advantage of using the Tempest et al. approach to construct NEWM and REI based humanized antibodies is that the three dimensional structures of NEWM and REI variable regions are known from x-ray crystallography and thus specific interactions between CDRs and V region framework residues can be modeled.

Non-human antibodies can be modified to include substitutions that insert human immunoglobulin sequences, e.g., consensus human amino acid residues at particular positions, e.g., at one or more (preferably at least five, ten, twelve, or all) of the following positions: (in the FR of the variable domain of the light chain) 4L, 35L, 36L, 38L, 43L, 44L, 58L, 46L, 62L, 63L, 64L, 65L, 66L, 67L, 68L, 69L, 70L, 71L, 73L, 85L, 87L, 98L, and/or (in the FR of the variable domain of the heavy chain) 2H, 4H, 24H, 36H, 37H, 39H, 43H, 45H, 49H, 58H, 60H, 67H, 68H, 69H, 70H, 73H, 74H, 75H, 78H, 91H, 92H, 93H, and/or 103H (according to the Kabat numbering). See, e.g., US Pat. No. 6,407,213.

Fully human monoclonal antibodies that bind to VLA-4 can be produced, e.g., using in vitro-primed human splenocytes, as described by Boemer et al., 1991, J. Immunol., 147, 86-95. They may be prepared by repertoire cloning as described by Persson et al., 1991, Proc. Nat. Acad. Sci. USA, 88: 2432-2436 or by Huang and Stollar, 1991, J. Immunol. Methods 141, 227-236; also US Pat. No. 5,798,230. Large nonimmunized human phage display libraries may also be used to isolate high affinity antibodies that can be developed as human therapeutics using standard phage technology (see, e.g., Vaughan et al, 1996; Hoogenboom et al. (1998) Immunotechnology 4:1-20; and Hoogenboom et al. (2000) Immunol Today 2:371-8; US 2003-0232333).

### Antibody Production

Antibodies can be produced in prokaryotic and eukaryotic cells. In one embodiment, the antibodies (e.g., scFv's) are expressed in a yeast cell such as *Pichia* (see, e.g., Powers et al. (2001) J Immunol Methods. 251:123-35), *Hanseula,* or *Saccharomyces.*

In one embodiment, antibodies, particularly full length antibodies, e.g., IgG's, are produced in mammalian cells. Exemplary mammalian host cells for recombinant expression include Chinese Hamster Ovary (CHO cells) (including dhfr- CHO cells, described in Urlaub and Chasin (1980) Proc. Natl. Acad. Sci. USA 77:4216-4220, used with a DHFR selectable marker, e.g., as described in Kaufman and Sharp (1982) Mol. Biol. 159:601-621), lymphocytic cell lines, e.g., NS0 myeloma cells and SP2 cells, COS cells, K562, and a cell from a transgenic animal, e.g., a transgenic mammal. For example, the cell is a mammary epithelial cell.

In addition to the nucleic acid sequence encoding the immunoglobulin domain, the recombinant expression vectors may carry additional nucleic acid sequences, such as sequences that regulate replication of the vector in host cells (e.g., origins of replication) and selectable marker genes. The selectable marker gene facilitates selection of host cells into which the vector has been introduced (see e.g., US Pat. Nos. 4,399,216,4,634,665 and 5,179,017). Exemplary selectable marker genes include the dihydrofolate reductase (DHFR) gene (for use in *dhfr-* host cells with methotrexate selection/amplification) and the *neo* gene (for G418 selection).

In an exemplary system for recombinant expression of an antibody (e.g., a full length antibody or an antigen-binding portion thereof), a recombinant expression vector encoding both the antibody heavy chain and the antibody light chain is introduced into *dhfr-* CHO cells by calcium phosphate-mediated transfection. Within the recombinant expression vector, the antibody heavy and light chain genes are each operatively linked to enhancer/promoter regulatory elements (e.g., derived from SV40, CMV, adenovirus and the like, such as a CMV enhancer/AdMLP promoter regulatory element or an SV40 enhancer/AdMLP promoter regulatory element) to drive high levels of transcription of the genes. The recombinant expression vector also carries a DHFR gene, which allows for selection of CHO cells that have been transfected with the vector using methotrexate selection/amplification. The selected transformant host cells are cultured to allow for expression of the antibody heavy and light chains and intact antibody is recovered from the culture medium. Standard molecular biology techniques are used to prepare the recombinant expression vector, to transfect the host cells, to select for transformants, to culture the host cells, and to recover the antibody from the culture medium. For example, some antibodies can be isolated by affinity chromatography with a Protein A or Protein G US Pat. No. 6,602,503 also describes exemplary methods for expressing and purifying a VLA-4 binding antibody.

Antibodies may also include modifications, e.g., modifications that alter Fc function, e.g., to decrease or remove interaction with an Fc receptor or with C1q, or both. For example, the human IgG1 constant region can be mutated at one or more residues, e.g., one or more of residues 234 and 237, e.g., according to the numbering in US Pat. No. 5,648,260. Other exemplary modifications include those described in US Pat. No. 5,648,260.

For some antibodies that include an Fc domain, the antibody production system may be designed to synthesize antibodies in which the Fc region is glycosylated. For example, the Fc domain of IgG molecules is glycosylated at asparagine 297 in the CH2 domain. This asparagine is the site for modification with biantennary-type oligosaccharides. This glycosylation participates in effector functions mediated by Feγ receptors and complement C1q (Burton and Woof (1992) Adv. Immunol. 51:1-84; Jefferis et al. (1998) Immunol. Rev. 163:59-76). The Fc domain can be produced in a mammalian expression system that appropriately glycosylates the residue corresponding to asparagine 297. The Fc domain can also include other eukaryotic post-translational modifications.

Antibodies can also be produced by a transgenic animal. For example, US Pat. No. 5,849,992 describes a method for expressing an antibody in the mammary gland of a transgenic mammal. A transgene is constructed that includes a milk-specific promoter and nucleic acid sequences encoding the antibody of interest, e.g., an antibody described herein, and a signal sequence for secretion. The milk produced by females of such transgenic mammals includes, secreted-therein, the antibody of interest, e.g., an antibody described herein. The antibody can be purified from the milk, or for some applications, used directly.

Antibodies can be modified, e.g., with a moiety that improves its stabilization and/or retention in circulation, e.g., in blood, serum, lymph, bronchoalveolar lavage, or other tissues, e.g., by at least 1.5,2, 5, 10, or 50 fold.

For example, a VLA-4 binding antibody can be associated with a polymer, e.g., a substantially non-antigenic polymer, such as a polyalkylene oxide or a polyethylene oxide. Suitable polymers will vary substantially by weight. Polymers having molecular number average weights ranging from about 200 to about 35,000 daltons (or about 1,000 to about 15,000, and 2,000 to about 12,500) can be used.

For example, a VLA-4 binding antibody can be conjugated to a water soluble polymer, e.g., a hydrophilic polyvinyl polymer, e.g. polyvinylalcohol or polyvinylpyrrolidone. A non-limiting list of such polymers include polyalkylene oxide homopolymers such as polyethylene glycol (PEG) or polypropylene glycols, polyoxyethylenated polyols, copolymers thereof and block copolymers thereof, provided that the water solubility of the block copolymers is maintained. Additional useful polymers include polyoxyalkylenes such as polyoxyethylene, polyoxypropylene, and block copolymers of polyoxyethylene and polyoxypropylene (Pluronics); polymethacrylates; carbomers; branched or unbranched polysaccharides that comprise the saccharide monomers D-mannose, D- and L-galactose, fucose, fructose, D-xylose, L-arabinose, D-glucuronic acid, sialic acid, D-galacturonic acid, D-mannuronic acid (e.g. polymannuronic acid, or alginic acid), D-glucosamine, D-galactosamine, D-glucose and neuraminic acid including homopolysaccharides and heteropolysaccharides such as lactose, amylopectin, starch, hydroxyethyl starch, amylose, dextrane sulfate, dextran, dextrins, glycogen, or the polysaccharide subunit of acid mucopolysaccharides, e.g. hyaluronic acid; polymers of sugar alcohols such as polysorbitol and polymannitol; heparin or heparon.

### Pharmaceutical Compositions

A VLA-4 binding agent, such as a VLA-4 binding antibody, (e.g., natalizumab) can be formulated as a pharmaceutical composition. Typically, a pharmaceutical composition includes a pharmaceutically acceptable carrier. As used herein, "pharmaceutically acceptable carrier" includes any and all solvents, dispersion media, coatings, antibacterial and antifungal agents, isotonic and absorption delaying agents, and the like that are physiologically compatible.

A "pharmaceutically acceptable salt" refers to a salt that retains the desired biological activity of the parent compound and does not impart any undesired toxicological effects (see *e.g.,* Berge, S.M., et al. (1977) J. Pharm. Sci. 66:1-19). Examples of such salts include acid addition salts and base addition salts. Acid addition salts include those derived from nontoxic inorganic acids, such as hydrochloric, nitric, phosphoric, sulfuric, hydrobromic, hydroiodic, and the like, as well as from nontoxic organic acids such as aliphatic mono- and dicarboxylic acids, phenyl-substituted alkanoic acids, hydroxy alkanoic acids, aromatic acids, aliphatic and aromatic sulfonic acids and the like. Base addition salts include those derived from alkaline earth metals, such as sodium, potassium, magnesium, calcium and the like, as well as from nontoxic organic amines, such as N,N'-dibenzylethylenediamine, N-methylglucamine, chloroprocaine, choline, diethanolamine, ethylenediamine, procaine and the like.

Natalizumab and other agents described herein can be formulated according to standard methods. Pharmaceutical formulation is a well-established art, and is further described in Gennaro (ed.), Remington: The Science and Practice of Pharmacy, 20th ed., Lippincott, Williams & Wilkins (2000) (ISBN: 0683306472); Ansel et al., Pharmaceutical Dosage Forms and Drug Delivery Systems, 7th Ed.., Lippincott Williams & Wilkins Publishers (1999) (ISBN: 0683305727); and Kibbe (ed.), Handbook of Pharmaceutical Excipients American Pharmaceutical Association, 3rd ed. (2000) (ISBN: 091733096X).

In one embodiment, natalizumab or another agent (e.g., another antibody) can be formulated with excipient materials, such as sodium chloride, sodium dibasic phosphate heptahydrate, sodium monobasic phosphate, and polysorbate 80. It can be provided, for example, in a buffered solution at a concentration of about 20 mg/ml and can be stored at 2-8°C. Natalizumab (ANTEGREN®) can be formulated as described on the manufacturer's label.

Pharmaceutical compositions may also be in a variety of other forms. These include, for example, liquid, semi-solid and solid dosage forms, such as liquid solutions (e.g., injectable and infusible solutions), dispersions or suspensions, tablets, pills, powders, liposomes and suppositories. The preferred form can depend on the intended mode of administration and therapeutic application. Typically compositions for the agents described herein are in the form of injectable or infusible solutions.

Such compositions can be administered by a parenteral mode (*e.g*., intravenous, subcutaneous, intraperitoneal, or intramuscular injection). The phrases "parenteral administration" and "administered parenterally" as used herein mean modes of administration other than enteral and topical administration, usually by injection, and include, without limitation, intravenous, intramuscular, intraarterial, intrathecal, intracapsular, intraorbital, intracardiac, intradermal, intraperitoneal, transtracheal, subcutaneous, subcuticular, intraarticular, subcapsular, subarachnoid, intraspinal, epidural and intrasternal injection and infusion.

Pharmaceutical compositions typically must be sterile and stable under the conditions of manufacture and storage. A pharmaceutical composition can also be tested to insure it meets regulatory and industry standards for administration.

The composition can be formulated as a solution, microemulsion, dispersion, liposome, or other ordered structure suitable to high drug concentration. Sterile injectable solutions can be prepared by incorporating an agent described herein in the required amount in an appropriate solvent with one or a combination of ingredients enumerated above, as required, followed by filtered sterilization. Generally, dispersions are prepared by incorporating an agent described herein into a sterile vehicle that contains a basic dispersion medium and the required other ingredients from those enumerated above. In the case of sterile powders for the preparation of sterile injectable solutions, the preferred methods of preparation are vacuum drying and freeze-drying that yields a powder of an agent described herein plus any additional desired ingredient from a previously sterile-filtered solution thereof. The proper fluidity of a solution can be maintained, for example, by the use of a coating such as lecithin, by the maintenance of the required particle size in the case of dispersion and by the use of surfactants. Prolonged absorption of injectable compositions can be brought about by including in the composition an agent that delays absorption, for example, monostearate salts and gelatin.

### Administration

A VLA-4 binding antibody can be administered to a subject, e.g., a human subject, by a variety of methods. For many applications, the route of administration is one of: intravenous injection or infusion, subcutaneous injection, or intramuscular injection. A VLA-4 binding antibody, such as natalizumab, can be administered as a fixed dose, or in a mg/kg dose, but preferably as a fixed dose. The antibody can be administered intravenously (IV) or subcutaneously (SC). Natalizumab is typically administered at a fixed unit dose of between 50-600 mg IV, e.g., every 4 weeks, or between 50-100 mg SC (e.g., 75 mg), e.g., at least once a week (e.g., twice a week). It can also be administered in a bolus at a dose of between 1 and 10 mg/kg, e.g., about 6.0, 4.0, 3.0, 2.0, 1.0 mg/kg. Modified dose ranges include a dose that is less than 600, 400, 300, 250, 200, or 150 mg/subject, typically for administration every fourth week or once a month. The VLA-4 binding antibody can administered, for example, every three to five weeks, e.g., every fourth week, or monthly.

The dose can also be chosen to reduce or avoid production of antibodies against the VLA-4 binding antibody, to achieve greater than 40, 50, 70, 75, or 80% saturation of the α4 subunit, to achieve to less than 80, 70, 60, 50, or 40% saturation of the α4 subunit, or to prevent an increase the level of circulating white blood cells

In certain embodiments, the active agent may be prepared with a carrier that will protect the compound against rapid release, such as a controlled release formulation, including implants, and microencapsulated delivery systems. Biodegradable, biocompatible polymers can be used, such as ethylene vinyl acetate, polyanhydrides, polyglycolic acid, collagen, polyorthoesters, and polylactic acid. Many methods for the preparation of such formulations are patented or generally *known. See, e.g.,* Sustained and Controlled Release Drug Delivery Systems, J.R. Robinson, ed., Marcel Dekker, Inc., New York, 1978.

Pharmaceutical compositions can be administered with medical devices. For example, pharmaceutical compositions can be administered with a needleless hypodermic injection device, such as the devices disclosed in US Pat. Nos. 5,399,163, 5,383,851, 5,312,335, 5,064,413, 4,941,880, 4,790,824, or 4,596,556. Examples of well-known implants and modules include: US Pat. No. 4,487,603, which discloses an implantable micro-infusion pump for dispensing medication at a controlled rate; US Pat. No. 4,486,194, which discloses a therapeutic device for administering medicants through the skin; US Pat. No. 4,447,233, which discloses a medication infusion pump for delivering medication at a precise infusion rate; US Pat. No. 4,447,224, which discloses a variable flow implantable infusion apparatus for continuous drug delivery; US Pat. No. 4,439,196, which discloses an osmotic drug delivery system having multichamber compartments; and US Pat. No. 4,475,196, which discloses an osmotic drug delivery system. Of course, many other such implants, delivery systems, and modules are also known.

This disclosure also features a device for administering a first and second agent. The device can include, e.g., one or more housings for storing pharmaceutical preparations, and can be configured to deliver unit doses of the first and second agent. The first and second agents can be stored in the same or separate compartments. For example, the device can combine the agents prior to administration. It is also possible to use different devices to administer the first and second agent.

Dosage regimens are adjusted to provide the desired response, e.g., a therapeutic response or a combinatorial therapeutic effect. Generally, any combination of doses (either separate or co-formulated) of the VLA-4 binding agent and the second agent can be used in order to provide a subject with both agents in bioavailable quantities.

Dosage unit form or "fixed dose" as used herein refers to physically discrete units suited as unitary dosages for the subjects to be treated; each unit contains a predetermined quantity of active compound calculated to produce the desired therapeutic effect in association with the required pharmaceutical carrier and optionally in association with the other agent.

A pharmaceutical composition may include a "therapeutically effective amount" of an agent described herein. Such effective amounts can be determined based on the combinatorial effect of the administered first and second agent. A therapeutically effective amount of an agent may also vary according to factors such as the disease state, age, sex, and weight of the individual, and the ability of the compound to elicit a desired response in the individual, e.g., amelioration of at least one disorder parameter, e.g., a multiple sclerosis parameter, or amelioration of at least one symptom of the disorder, e.g., multiple sclerosis. A therapeutically effective amount is also one in which any toxic or detrimental effects of the composition is outweighed by the therapeutically beneficial effects.

### Exemplary Second Agents

In certain embodiments, a subject who has severe multiple sclerosis can be administered a second agent, in combination with a VLA-4 binding antibody. Non-limiting examples of agents for treating or preventing multiple sclerosis that can be administered with a VLA-4 binding antibody include the following exemplary second agents:
■ interferons, e.g., interferon beta, e.g., human interferon-beta-1a (e.g., AVONEX® or Rebif®)) and interferon-1β (BETASERON™; human interferon β substituted at position 17; Berlex/Chiron);
■ glatiramer acetate (also termed Copolymer 1, Cop-1; COPAXONE™; Teva Pharmaceutical Industries, Inc.);
■ fumarates, e.g., dimethyl fumarate (e.g., Fumaderm®);
■ Rituxan® (rituximab) or another anti CD20 antibody, e.g., one that competes with or binds an overlapping epitope with rituximab;
■ mixtoxantrone (NOVANTRONE®, Lederle);
■ a chemotherapeutic, e.g., clabribine (LEUSTATIN®), azathioprine (IMURAN®), cyclophosphamide (CYTOXAN®), cyclosporine-A, methotrexate, 4-aminopyridine, and tizanidine;
■ a corticosteroid, e.g., methylprednisolone (MEDRONE®, Pfizer), prednisone;
■ an immunoglobulin, e.g., Rituxan® (rituximab); CTLA4 Ig; alemtuzumab (MabCAMPATH®) or daclizumab (an antibody that binds CD25);
■ statins;
■ azathioprine; and
■ TNF antagonists.

Other exemplary second agents and methods for administering them in combination with a VLA-4 binding antibody are described in U.S. Serial No. 60/603,468.

All patent applications, patents, references and publications included herein are incorporated herein by reference.

Other embodiments are within the scope of the following claims.

Furthermore, the present invention relates to the following items:
1. A method of treating a subject having multiple sclerosis (MS), the method comprising administering, to the subject, a therapeutically effective amount of a VLA-4 binding antibody for at least 24 months.
2. A method of treating a subject having MS, the method comprising:
   selecting a patient who has received at least 22 doses of a VLA-4 binding antibody over a period of 24 months; and
   administering to the patient at least one course of VLA-4 binding antibody therapy sufficient to provide an improved therapeutic effect compared to before commencement of VLA-4 binding antibody therapy.
3. The method of item 2, wherein the course comprises at least 3 administrations of a therapeutically effective amount of a VLA-4 binding antibody.
4. The method of item 3, wherein the administrations are monthly administrations.
5. The method of item 3, wherein the administrations are monthly IV administrations.
6. The method of item 1, wherein the VLA-4 binding antibody is administered for at least 30 months.
7. The method of item 1, wherein the VLA-4 binding antibody is administered for at least 36 months.
8. The method of item 1, wherein the VLA-4 binding antibody is administered for at least 48 months.
9. The method of item 1, wherein the subject is administered the VLA-4 binding antibody according to a regimen that is effective to achieve at least 50% alpha4 integrin receptor saturation in the subject.
10. The method of item 1, wherein the subject is administered a plurality of doses of the VLA-4 binding antibody intravenously, wherein each dose is between 200 and 400 mg.
11. The method of item 2, wherein the course comprises a plurality of doses of the VLA-4 binding antibody intravenously, wherein each dose is between 200 and 400 mg.
12. The method of item 1, wherein the VLA-4 binding antibody is administered in combination with a second therapeutic agent.
13. The method of item 1, wherein the subject has a type of multiple sclerosis selected from the group consisting of chronic progressive multiple sclerosis, primary-progressive (PP) multiple sclerosis, secondary progressive multiple sclerosis, and progressive relapsing multiple sclerosis.
14. The method of item 1, wherein the VLA-4 binding antibody inhibits VLA-4 interaction with VCAM-1.
15. The method of item 1, wherein the VLA-4 binding antibody is natalizumab.
16. The method of item 1, wherein the VLA-4 binding antibody is human or humanized.
17. The method of item 1, wherein the VLA-4 binding antibody competes with HP1/2 or natalizumab for binding to VLA-4.

## Claims

1. Use of a VLA4 binding antibody for the preparation of a medicament for treating a subject having multiple sclerosis (MS), wherein said treatment comprises administering the medicament to the subject in an amount effective to achieve less than 60%, less than 50% or less than 40% saturation of the α4 subunit of VLA4.

2. Use of a VLA4 binding antibody for the preparation of a medicament for treating a subject having multiple sclerosis (MS), wherein said treatment comprises administering the medicament to the subject in combination with a fumarate.

3. The use of claim 2, wherein said fumarate is dimethyl fumarate.

4. The use of claim 2 or 3, wherein the medicament is administered in an amount effective to achieve less than 60%, less than 50% or less than 40% saturation of the α4 subunit of VLA4 in the subject.

5. A VLA4 binding antibody for use in treating a subject having multiple sclerosis (MS),
wherein said treatment comprises administering the VLA4 binding antibody in an amount effective to achieve less than 60%, less than 50% or less than 40% saturation of the α4 subunit of VLA4.

6. A VLA4 binding antibody for use in treating a subject having multiple sclerosis (MS),
wherein said treatment comprises administering the VLA4 binding antibody in combination with a fumarate.

7. The VLA4 binding antibody for use according to claim 6, wherein said fumarate is dimethyl fumarate.

8. The VLA4 binding antibody for use according to claim 6 or 7, wherein the VLA4 binding antibody is administered in an amount effective to achieve less than 60%, less than 50% or less than 40% saturation of the α4 subunit of VLA4 in the subject.

9. The use of any one of claims 1 to 4 or the VLA4 binding antibody for use according to any one of claims 5 to 8, wherein the medicament or VLA4 binding antibody is administered for at least 12, 18, 24, 30, 36, 42, or 48 months.

10. The use of any one of claims 1 to 4 or the VLA4 binding antibody for use according to any one of claims 5 to 8, wherein said treatment comprises administering a corticosteroid within 5, 10, 30, or 60 days prior to administration of the medicament or VLA4 binding antibody.
